# EUROPÄISCHE PATENTSCHRIFT

(11) **EP 3 139 992 B1**
(45) Veröffentlichungstag und Bekanntmachung des Hinweises auf die Patenterteilung: **06.02.2019**
(21) Anmeldenummer: 15738242.5
(22) Anmeldetag: 30.04.2015
(51) Int. Cl.: A61M 39/16, A61M 39/10, A61M 39/00, A61M 5/165

(54) **VERBINDUNGSEINRICHTUNG FÜR EINEN KATHETER, INSBESONDERE EINEN PERIPHEREN VENENKATHETER (II)**
CONNECTOR DEVICE FOR A CATHETER, PARTICULARLY A PERIPHERAL VENOUS CATHETER (II)
MOYEN DE CONNEXION POUR UN CATHÉTER, EN PARTICULIER UN CATHÉTER VEINEUX PÉRIPHÉRIQUE (II)

(30) Priorität: 08.05.2014 DE 102014106454
(43) Veröffentlichungstag der Anmeldung: 15.03.2017
(73) Patentinhaber: SICOS GmbH, 40229 Düsseldorf (DE)
(72) Erfinder: REICHARDT, André, 99636 Rastenberg/Thürigen (DE); FRAUENRATH, Christian, 52428 Jülich (DE); MARKUS, Kai, 52249 Eschweiler (DE)
(74) Vertreter: Naeven, Ralf
(86) Internationale Anmeldenummer: PCT/DE2015/100178
(87) Internationale Veröffentlichungsnummer: WO 2015/169283

(56) Entgegenhaltungen:
- EP-A1- 1 057 495
- EP-B1- 1 579 887
- WO-A1-95/21648
- DE-A1-102012 108 791
- US-A- 5 176 415
- US-A- 5 190 534
- US-A- 5 190 534
- US-A- 5 782 808
- US-A1- 2006 015 086
- US-A1- 2008 264 450
- US-A1- 2013 131 583

## Beschreibung

Die Erfindung betrifft eine Verbindungseinrichtung für einen Katheter, insbesondere einen peripheren Venenkatheter umfassend einen Hauptkörper mit einem Hauptkörper-Infusionskanal und einem sterilisierend wirkenden und zumindest einen Teil des Hauptkörper-Infusionskanals bildenden Durchflusselement, wobei das Durchflusselement eine Eingangsöffnung, eine flüssigkeitsdichte Umfangswand und eine der Eingangsöffnung gegenüberliegende Endwand mit mindestens einer Auslassöffnung aufweist, und einen einen Anschlusskörper-Infusionskanal aufweisenden und lösbar am Hauptkörper fixierten Anschlusskörper.

Eine Verbindungseinrichtung für einen peripheren Venenkatheter ist aus der EP 1 579 878 B1 bekannt, welche insbesondere bei zentralen Venenkathetern, die über lange Zeiträume am menschlichen Körper verbleiben, geeignet ist, die Gefahr von durch den Eintritt von Bakterien verursachten Sepsen zu verringern. Zentrale Venenkatheter können z. B. zur künstlichen Ernährung bei Kurzdarmsyndrom eingesetzt werden. Derartige Sepsen sind für die behandelte Person mit erheblichen persönlichen Nachteilen verbunden. Als Lösung schlägt der genannte Stand der Technik vor, als sterilisierendes Durchflusselement ein zahlreiche Öffnungen aufweisendes Siebelement aus Silber vorzusehen, welches im Infusionskanal oder in Infusionsflussrichtung gesehen am Ende des Infusionskanals angeordnet ist. Das Siebelement stellt eine Barriere für Bakterien und/oder Pilze dar und ist auch deshalb vorteilhaft, weil die komplette Verbindungseinrichtung zusammen mit dem Siebelement auf sehr einfache Art und Weise ausgetauscht werden kann, wenn das Siebelement z. B. in Folge einer Verstopfung unwirksam werden sollte.

Aus der US 5,782,808 ist eine Verbindungseinrichtung der eingangs genannten Art bekannt. Dort ist der das Durchflusselement umfassende Hauptkörper einstückig. Es ist des Weiteren offenbart, diverse Flächen des Hauptkörpers wie auch Flächen des mit dem Hauptkörper über ein Gewinde verbindbaren Anschlusskörpers mit einer sterilisierend wirkenden Beschichtung zu versehen. In einer Ausgestaltung ist zudem die Möglichkeit offenbart, auch die Innenflächen des Durchflusselements mit der Silberbeschichtung zu versehen. Für die Abdichtung zwischen Hauptkörper und Anschlusskörper sind Dichtungsringe vorgesehen.

Aus der US 5,049,139 ist eine Verbindungseinrichtung für einen Katheter bekannt, bei dem zwischen zwei Teilen der Verbindungseinrichtung ein einen Durchlass für die Infusionsflüssigkeit aufweisenden Block aus einem wasserlöslichen Glas angeordnet ist, welches mit Silber dotiert ist. Beim Einsatz lösen sich Teile des Blocks in der Infusionsflüssigkeit, so dass die antibakteriellen Eigenschaften des Silbers zur Wirkung kommen können. Dabei ist auch offenbart, dass der eingesetzte Glasblock einen ringförmigen Flansch aufweist, welcher zwischen den beiden Teilen der Verbindungseinrichtung eingeklemmt wird. Eine Dichtfunktion am Ort des ringförmigen Flanschs ist nicht genannt. Aufgrund der Löslichkeit des Glasblocks in der Infusionsflüssigkeit dürfte eine solche Dichtfunktion nicht gegeben sein, weshalb andere Dichtungsmaßnahmen erforderlich sind. Die Abdichtungsproblematik wird nicht angesprochen.

Die EP 1057495 A1 offenbart eine für einen Katheter geeignete Verbindungsvorrichtung mit einem Durchflusselement, welches an seinem vorderen Ende konusförmig ausgebildet ist und mit einem Anschlusskörper eine Presspassung eingeht. Eine sterilisierende Wirkung des Durchflusselements ist nicht angesprochen.

Es ist nun Aufgabe der vorliegenden Erfindung, eine Verbindungseinrichtung der eingangs genannten Art zur Verfügung zu stellen, welche einfach herstellbar ist und die Schutzwirkung gegenüber Sepsen weiter verbessern kann.

Diese Aufgabe wird bei einer Verbindungseinrichtung der eingangs genannten Art durch die kennzeichnenden Merkmale des Anspruchs 1 gelöst.

Beispielhafte und vorteilhafte Ausbildungsformen der Verbindungseinrichtung sind in den abhängigen Ansprüchen definiert.

Das Durchflusselement ist in der Umfangswand undurchlässig für eine Flüssigkeit, insbesondere für eine Infusionsflüssigkeit, die damit allein durch die mindestens eine Auslassöffnung der Endwand hindurch in den Anschlusskörper-Infusionskanal eintreten kann. Mit der dichtenden Presspassung zwischen Durchflusselement und Anschlusskörper-Infusionskanal kann die Dichtigkeit der Verbindung zwischen dem Hauptkörper und dem Anschlusskörper in Bezug auf die Infusionsflüssigkeit gewährleistet werden.

Hauptkörper und Anschlusskörper können z.B. mit Gewinden aneinander fixiert sein. Es kann zusätzlich zur dichtenden Presspassung zwischen Durchflusselement und Anschlusskörper-Infusionskanal eine dichtende Presspassung zwischen Hauptkörper und Anschlusskörper vorgesehen sein.

Mit der dichtenden Presspassung zwischen Durchflusselement und Anschlusskörper-Infusionskanal können weitere Dichtungsmaßnahmen unterstützt oder auch entbehrlich werden. Mit der Presspassung können Fertigungstoleranzen bei den Abmessungen des Anschlusskörper-Infusionskanals zu einem gewissen Maß aufgefangen werden.

Zum Erreichen der Presspassung weisen das Durchflusselement und die Innenwand des Anschlusskörper-Infusionskanals geeignete Formen auf. Beispielsweise kann sich die Innenwand des Anschlusskörper-Infusionskanals in Flussrichtung der Infusionsflüssigkeit verjüngen, beispielsweise kann die Innenwand zumindest im Bereich des vorgesehenen Kontaktes mit dem sterilisierenden Durchflusselement konisch mit einem bestimmten Öffnungswinkel geformt sein. In diesem Fall könnte das Durchflusselement zumindest im Bereich des zu erwartenden Kontaktes mit der Innenwand des Anschlusskörper-Infusionskanals eine zylindrisch geformte Außenwand aufweisen oder sich ebenfalls in Flussrichtung verjüngen, wobei die pro Längeneinheit gegebene Durchmesserverringerung der Außenseite der Umfangswand des Durchflusselements geringer ist, als die pro Längeneinheit gegebene Durchmesserverringerung der Innenwand des Anschlusskörper-Infusionskanals. Im Falle einer Konusform weist die Außenseite der Umfangswand des Durchflusselements einen geringeren Öffnungswinkel als die Innenwand des Anschlusskörper-Infusionskanals auf.

Für die Presspassung vorteilhaft kann die Wahl eines elastischen Materials für das Durchflusselement und/oder für den Anschlusskörper-Infusionskanal sein.

Das Durchflusselement kann in seiner Endwand eine einzelne Auslassöffnung oder zwei oder mehr Auslassöffnungen aufweisen. Es kann auch eine Vielzahl von kleinen Auslassöffnungen vorgesehen sein, womit das Durchflusselement einen siebartigen Aufbau erhält.

Das Durchflusselement kann im Hauptkörper fixiert sein. Vorzugsweise ist die Fixierung des Durchflusselements für eine leichte Austauschbarkeit und/oder für einen leichteren Zugang zu Reinigungszecken lösbar ausgestaltet.

Es kann aber auch vorteilhaft sein, das erfindungsgemäße Verbindungselement so auszugestalten, dass der das Durchflusselement umfassende Hauptkörper einstückig ist. Dabei kann es vorteilhaft sein, wenn der Hauptkörper ein sterilisierend wirkendes Massivmaterial umfasst, insbesondere gänzlich daraus besteht. Unter einem Massivmaterial wird ein Material verstanden, welches ohne Auftrag auf einen Träger oder ein tragendes Substrat eine Formstabilität aufweist. Folien oder auf ein Substrat aufgetragene dünne Schichten sind somit kein Massivmaterial. Ein Massivmaterial kann jedoch eine mit Fremdstoffen dotierte oder Fremdelemente, z.B. Verstärkungselemente, insbesondere Fasern, enthaltende Matrix umfassen.

Mit der Fertigung des einstückigen Hauptkörpers aus einem sterilisierend wirkenden Massivmaterial kann sich eine Beschichtung, insbesondere eine solche mit sterilisierender Wirkung, erübrigen. Das Massivmaterial kann z.B. gänzlich aus einem einzelnen Stoff mit einem sterilisierend wirkenden Bestandteil, bestehen. Alternativ kann das Massivmaterial eine Matrix aus mindestens einem Material umfassen, in welche mindestens eine sterilisierend wirkende Substanz eingearbeitet ist, z.B. in Form eines Additivs oder einer Dotierung. Das mindestens eine Matrixmaterial, das selbst ohne oder mit sterilisierender Wirkung sein kann, kann z.B. ein Kunststoff sein. Insbesondere vorteilhaft ist es, wenn der gesamte Hauptkörper aus einem einheitlichen Kunststoffmaterial als Matrix mit einer sterilisierend wirkenden Dotierung besteht.

Als sterilisierend wirkend werden insbesondere solche Stoffe und Substanzen verstanden, welche insbesondere bakterizid, antibiotisch und/oder zytostatisch sind. Als sterilisierend wirkender Stoff oder als sterilisierend wirkende Substanz können z.B. Silber, Kupfer oder Silber und/oder Kupfer enthaltende Legierungen oder Silber und/oder Kupfer enthaltende chemische Verbindungen eingesetzt werden.

Die erfindungsgemäße Verbindungseinrichtung ist so ausgebildet, dass im Inneren des Durchflusselements mindestens ein sterilisierend wirkender Einsatzkörper angeordnet ist. Ein solcher Einsatzkörper vergrößert die sterilisierende Oberfläche und kann unabhängig von der Anzahl der Auslassöffnungen eingesetzt werden. Der mindestens eine Einsatzkörper kann zum Beispiel in Durchflussrichtung gesehen einen kreuzförmigen Querschnitt aufweisen. Der mindestens eine Einsatzkörper im Inneren des Durchflusselements kann gesondert und auswechselbar oder ebenfalls einstückig mit dem Durchflusselement oder Teil des einstückigen Hauptkörpers sein.

Der mindestens eine Einsatzkörper kann aus demselben Material wie der Hauptkörper bestehen. Der Einsatzkörper oder mindestens einer der Einsatzkörper kann aber auch aus einem anderen Material als dem des Hauptkörpers bestehen. Als Material des mindestens einen Einsatzkörpers kommt z.B. massives Silber oder Kupfer oder eine massive Silber und/oder Kupfer enthaltende Legierung oder ein sterilisierend dotierter Kunststoff in Frage, wobei im Falle mehrerer Einsatzkörper diese auch aus unterschiedlichen Materialien bestehen können

Das Durchflusselement und/oder der sterilisierend wirkende Einsatzkörper können z. B. aus Silber bestehen oder an der Oberfläche mit Silber beschichtet sein. Anstelle von Silber kann z.B. auch eine silber- oder kupferhaltige Legierung oder eine silber- oder kupferhaltige chemische Verbindung oder ein sonstiges sterilisierend wirkendes Material eingesetzt werden, z.B. ein Kunststoff mit bakteriziden, antibiotischen und/oder zytostatischen Additiven. Das Additiv kann beispielsweise Silber oder Kupfer sein. Durchflusselement und Einsatzkörper können auch aus unterschiedlichen Materialien bestehen.

Der Anschlusskörper-Infusionskanal kann ebenfalls eine sterilisierende Wirkung aufweisen, z.B. durch eine geeignete Beschichtung oder in die den Anschlusskörper-Infusionskanal begrenzende Wand eingearbeitete sterilisierend wirkende Substanzen, z. B. in Form einer Dotierung. Dadurch wird die sterilisierende Oberfläche, welche mit der durchströmenden Flüssigkeit in unmittelbarem Kontakt steht, vergrößert, was einen erhöhten Schutz bedeuten kann. Insbesondere kann der Anschlusskörper ein sterilisierend wirkendes Massivmaterial umfassen, wobei sämtliche oben zum Hauptkörper beschriebenen Materialvarianten des Massivmaterials auch für den Anschlusskörper vorteilhaft sein können. Außerdem kann der Anschlussköper einstückig ausgebildet sein.

Die erfindungsgemäße Verbindungseinrichtung kann auch so ausgebildet sein, dass der Anschlusskörper an seinem dem Hauptkörper abgewandten Ende ein Koppelstück für den Anschluss an eine Kanüle, insbesondere an eine Flügelkanüle aufweist. Hierdurch kann das Anlegen eines Katheters beschleunigt und vereinfacht werden. Zwischenelemente, z.B. in Schlauchform, welche zudem kontaminiert sein können und deren Anschluss Zeit kostet, werden hierdurch verzichtbar.

Es kann aber auch vorteilhaft sein, die erfindungsgemäße Verbindungseinrichtung so auszugestalten, dass der Anschlusskörper Teil einer Kanüle, insbesondere einer Flügelkanüle ist.

Im Folgenden wird eine bevorzugte Ausführungsform der erfindungsgemäßen Verbindungseinrichtung anhand von Figuren vorgestellt.

Es zeigt:
- Fig. 1:: eine erste Ausführungsform einer Verbindungseinrichtung mit im Hauptkörper fixiertem Durchflusselement,
- Fig. 2:: ein Durchflusselement mit zahlreichen Auslassöffnungen,
- Fig. 3:: ein Durchflusselement mit sterilisierendem Einsatzkörper in perspektivischer Ansicht,
- Fig. 4:: das Durchflusselement nach Fig. 3 in Aufsicht und
- Fig. 5:: eine zweite Ausführungsform einer Verbindungseinrichtung mit einstückigem Hauptkörper.

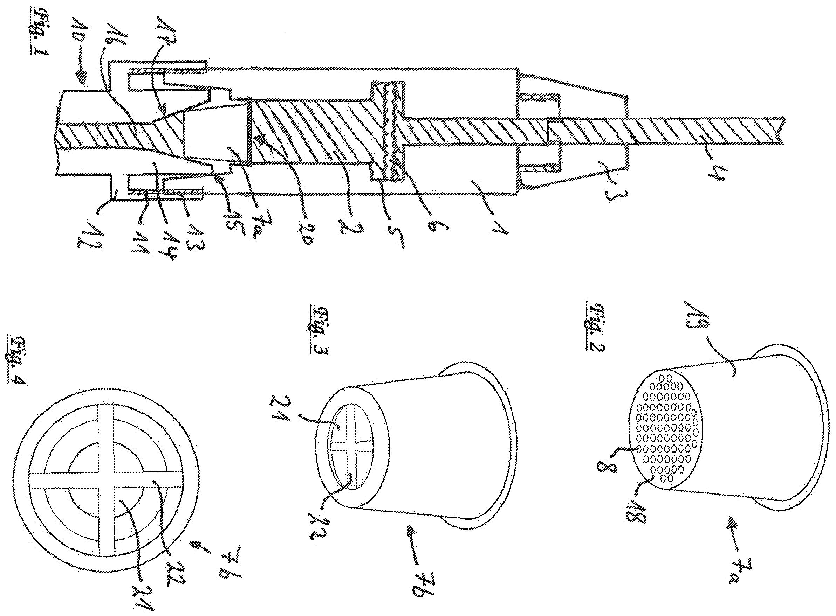

In schematischer Darstellung zeigt Fig. 1 eine erste Verbindungseinrichtung mit einem Hauptkörper 1, durch den ein Hauptkörper-Infusionskanal 2 verläuft. Der im Betrieb von Infusionsflüssigkeit durchsetzte Bereich ist in Fig. 1 schraffiert dargestellt. Der Hauptkörper 1 ist über ein Verbindungselement 3 an einen Infusionsschlauch 4 gekoppelt. Im Hauptkörper-Infusionskanal 2 ist eine Aufweitung 5 vorgesehen, welche einen Bakterienfilter 6 aufweist.

Am unteren Ende des Hauptkörper-Infusionskanals 2 ist ein Durchflusselement 7a fixiert, welches in Fig. 2 vergrößert in einer perspektivischen Ansicht von schräg unten dargestellt ist. Das Durchflusselement 7a weist allein an einer Endwand 18 zahlreiche Auslassöffnungen 8 auf, während eine Umfangswand 19 flüssigkeitsdicht gestaltet ist. Die Endwand 18 kann - wie dargestellt - flach sein oder eine beliebige andere, z.B. auch bauchige, Form aufweisen. Das Durchflusselement 7a besteht aus einem sterilisierenden Material und/oder ist mit einem sterilisierenden Material beschichtet. Das sterilisierende Material kann zum Beispiel Silber oder Kupfer sein. Die sterilisierende Wirkung des Durchflusselements 7a kann hinreichend sein, um auf den dargestellten Bakterienfilter 6 verzichten zu können.

Ein Anschlusskörper 10 ist mittels eines ein Innengewinde 11 aufweisenden Mutterelementes 12 an einem Außengewinde 13 des Hauptkörpers 1 fixiert. Der Anschlusskörper 10 weist einen Eingriffsstutzen 14 auf, der in einen Endbereich 15 des Hauptkörper-Infusionskanals 2 eingreift. Der Eingriffsstutzen 14 ist aus einem flexiblen Material und relativ zum Endbereich 15 des Hauptkörpers 1 derart dimensioniert, dass es bei der Fixierung des Anschlusskörpers 10 zu einer Presspassung zwischen dem Eingriffsstutzen 14 und dem Endbereich 15 des Hauptkörpers 1 kommt.

Bei fixiertem Anschlusskörper 10 ragt das Durchflusselement 7a in einen Anschlusskörper-Infusionskanal 16 hinein, welcher in einem Eingangsbereich 17 aufgeweitet ist.

Die Umfangswand 19 des Durchflusselements 7a ist konisch geformt, weist aber einen deutlich geringeren Öffnungswinkel auf als die Innenwand des ebenfalls konischen Eingriffsstutzens 14. Mit der Verbindung zwischen Hauptkörper 1 und Anschlusskörper 10 kommt es zu einer Presspassung zwischen dem Durchflusselement 7a und dem Eingriffsstutzen 14. Mit der Presspassung ist eine Dichtfunktion gegeben. Eine Dichtfunktion in der Verbindung zwischen Anschlusskörper 10 und Hauptkörper 1 ist somit nicht mehr erforderlich oder wird zumindest durch die Presspassung zwischen dem Durchflusselement 7a und dem Anschlusskörper-Infusionskanal 16 unterstützt.

Fig. 3 in perspektivischer Sicht und Fig. 4 in einer Aufsicht zeigen ein Durchflusselement 7b, welches lediglich eine einzige Auslassöffnung 21 aufweist und in dem gemäß der Erfindung ein sterilisierend wirkender kreuzförmiger Einsatzkörper 22 angeordnet ist.

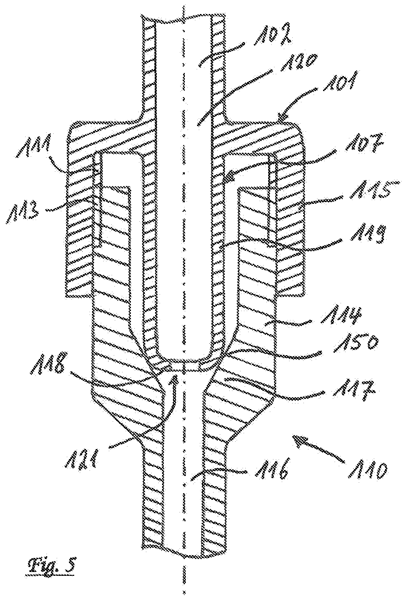

Fig. 5 zeigt eine zweite Verbindungseinrichtung mit einem einen Hauptkörper-Infusionskanal 102 aufweisenden Hauptkörper 101. In Fig. 5 ist - anders als in Fig. 1 - das Massivmaterial der beteiligten Körper schraffiert dargestellt. Der Hauptkörper 101 ist in hier nicht dargestellter Weise an einen hier ebenfalls nicht gezeigten zuführenden Infusionsschlauch angeschlossen. Der ein Durchflusselement 107 umfassende Hauptkörper 101 ist einstückig ausgebildet. Das Durchflusselement 107 weist eine Eingangsöffnung 120 und eine flüssigkeitsdichte Umfangswand 119 auf und bildet einen Teil des Infusionskanals 102 des Hauptkörpers 101.

Über eine ein Gewinde 111 aufweisende Koppelhülse 115, welche ein Teilstück des Durchflusselements 107 umgibt, kann ein einen Anschlusskörper-Infusionskanal 116 aufweisender Anschlusskörper 110 am Hauptkörper 101 fixiert werden. Hierfür wird eine ein Gewinde 113 aufweisende Eingriffshülse 114 des Anschlusskörpers 110 in die Koppelhülse 115 eingedreht. Ein Endstück 150 des Durchflusselements 107 geht beim Eindrehen des Anschlusskörpers 110 mit einem konusförmigen Eingangstück 117 des Anschlusskörpers 110 eine flüssigkeitsdichte Presspassung ein. Die untere Endwand 118 des Durchflusselements 107 weist eine Auslassöffnung 121 für den Durchfluss der Infusionsflüssigkeit auf. Alternativ kann die Endwand 118 auch eine Mehrzahl von Öffnungen, insbesondere eine Vielzahl von Sieböffnungen aufweisen, wie sie an der Endwand 18 des Durchflusselements 7a der Fig. 2 gezeigt ist.

Der Anschlusskörper 110 ist kann an einem hier nicht dargestellten abführenden Infusionsschlauch oder an eine hier ebenfalls nicht dargestellte Kanüle angeschlossen sein. Der Anschlusskörper 110 kann auch Teil der Kanüle sein.

Das Durchflusselement 107 kann auch mindestens einen in Fig. 5 nicht dargestellten Einsatzkörper aufweisen, der ebenfalls eine sterilisierende Wirkung haben kann. Der Einsatzkörper könnte beispielsweise in Flussrichtung einen kreuzförmigen Querschnitt aufweisen, entsprechend dem in Fig. 3 und 4 gezeigten Einsatzkörper. Andere Formen des Einsatzkörpers sind ebenfalls möglich. Der Einsatzkörper kann gesondert, insbesondere lösbar sein. Der Einsatzkörper kann aber auch einstückig mit dem Durchflusselement 107 und damit mit dem restlichen Hauptkörper 101 sein.

Der Hauptkörper 101 mit Durchflusselement 107 ist aus einem sterilisierend wirkenden Massivmaterial, z.B. umfassend Silber oder Kupfer oder einen Kunststoff mit einem z.B. in Form von Einschlüssen oder einer Dotierung eingearbeiteten sterilisierend wirkenden Material, z.B. Silber oder Kupfer, gefertigt.

**Bezugszeichenliste**

| | | | |
|---|---|---|---|
| 1. | Hauptkörper | 114. | Eingriffshülse |
| 2. | Hauptkörper-Infusionskanal | 115. | Koppelhülse |
| 3. | Verbindungselement | 117. | Kontaktstück |
| 4. | Infusionsschlauch | 118. | Endwand |
| 5. | Aufweitung | 119. | Umfangswand |
| 6. | Bakterienfilter | 121. | Auslassöffnung |
| 7. | Durchflusselement | | |
| 8. | Sieböffnung | | |
| 10. | Anschlusskörper | | |
| 11. | Innengewinde | | |
| 12. | Mutterelement | | |
| 13. | Außengewinde | | |
| 14. | Eingriffsstutzen | | |
| 15. | Endbereich | | |
| 16. | Anschlusskörper-Infusionskanal | | |
| 17. | Eingangsbereich | | |
| 18. | Endwand | | |
| 19. | Umfangswand | | |
| 20. | Eingangsöffnung | | |
| 21. | Auslassöffnung | | |
| 22. | Einsatz | | |
| 101. | Hauptkörper | | |
| 102. | Hauptkörper-Infusionskanal | | |
| 107. | Durchflusselement | | |
| 110. | Anschlusskörper | | |
| 111. | Gewinde | | |
| 113. | Gewinde | | |

## Patentansprüche

1. Verbindungseinrichtung für einen Katheter, insbesondere einen peripheren Venenkatheter, umfassend
a) einen Hauptkörper (1, 101) mit einem Hauptkörper-Infusionskanal (2, 102) und einem sterilisierend wirkenden und zumindest einen Teil des Hauptkörper-Infusionskanals (2, 102) bildenden Durchflusselement (7b, 107), wobei das Durchflusselement (7b, 107) eine Eingangsöffnung (20, 120) und eine flüssigkeitsdichte Umfangswand (19, 119) aufweist, und
b) einen einen Anschlusskörper-Infusionskanal (16, 116) aufweisenden und lösbar am Hauptkörper (1, 101) fixierten Anschlusskörper (10, 110),
**dadurch gekennzeichnet, dass**
c) das Durchflusselement (7b, 107) bei am Hauptkörper (1, 101) fixiertem Anschlusskörper (10, 110) unmittelbar mit dem Anschlusskörper-Infusionskanal (16, 116) eine dichtende Presspassung eingeht,
d) das Durchflusselement (7b, 107) eine der Eingangsöffnung (20, 120) gegenüberliegende Endwand (18, 118) mit mindestens einer Auslassöffnung (8, 21, 121) aufweist und
e) im Inneren des Durchflusselements (7b, 107) mindestens ein sterilisierend wirkender, den in Durchflussrichtung gegebenen Querschnitt des Hauptkörper-Infusionskanals durchquerender Einsatzkörper (22) angeordnet ist.

2. Verbindungseinrichtung nach Anspruch 1, **dadurch gekennzeichnet, dass** sich der Anschlusskörper-Infusionskanal (16, 116) zumindest in einem ein Endstück des Durchflusselements (7b, 107) umgebenden Eingangsstück (17, 117) in Flussrichtung verjüngt.

3. Verbindungseinrichtung nach einem der vorhergehenden Ansprüche, **dadurch gekennzeichnet, dass** die Außenseite der Umfangswand (19, 119) des Durchflusselements (7b, 107) zylindrisch ist.

4. Verbindungseinrichtung nach einem der Ansprüche 1 oder 2, **dadurch gekennzeichnet, dass** die Außenseite der Umfangswand (19, 119) des Durchflusselements (7b, 107) konisch mit zur Endwand (18, 118) hin abnehmendem Durchmesser ist.

5. Verbindungseinrichtung nach einem der vorhergehenden Ansprüche, **dadurch gekennzeichnet, dass** der Anschlusskörper (10, 110) zumindest auf einer Teilstrecke im Anschlusskörper-Infusionskanal (16, 116) eine sterilisierende Wirkung aufweist.

6. Verbindungseinrichtung nach einem der vorhergehenden Ansprüche, **dadurch gekennzeichnet, dass** der Hauptkörper (1, 101) einstückig ist.

7. Verbindungseinrichtung nach einem der vorhergehenden Ansprüche, **dadurch gekennzeichnet, dass** das Durchflusselement (7b, 107) aus einem Massivmaterial mit sterilisierender Wirkung bestehen.

8. Verbindungseinrichtung nach einem der vorhergehenden Ansprüche, **dadurch gekennzeichnet, dass** der gesamte Hauptkörper (1, 101) aus einem Massivmaterial mit sterilisierender Wirkung besteht.

9. Verbindungseinrichtung nach Anspruch 7 oder 8, **dadurch gekennzeichnet, dass** das Massivmaterial eine Matrix aus einem Kunststoff umfasst, wobei die Matrix mit einer sterilisierend wirkenden Substanz dotiert ist.

10. Verbindungseinrichtung nach einem der vorhergehenden Ansprüche, **dadurch gekennzeichnet, dass** der Anschlusskörper (10, 110) an seinem dem Hauptkörper (1, 101) abgewandten Ende ein Koppelstück für den Anschluss an eine Kanüle, insbesondere an eine Flügelkanüle, aufweist.

11. Verbindungseinrichtung nach einem der Ansprüche 1 bis 9, **dadurch gekennzeichnet, dass** der Anschlusskörper (10, 110) Teil einer Kanüle, insbesondere einer Flügelkanüle, ist.

## Claims

1. Connector device for a catheter, especially for a peripheral venous catheter, comprising
a) a main body (1, 101) with a main body infusion channel (2, 102) and through-flow element (7b, 107) having sterilizing effect and forming at least a part of the main body infusion channel (2, 102), wherein the through-flow element (7b, 107) has an inlet opening (20, 120) and a liquid-tight circumferential wall (19, 119), and
b) an attachment body (10, 110) having an attachment body infusion channel (16, 116) and being detachably fixed on the main body (1, 101),
**characterized in that**
c) the through-flow element (7b, 107) undergoes a sealing press fit with the attachment body infusion channel (16, 116) when the attachment body (10, 110) is fixed to the main body (1, 101),
d) the through-flow element (7b, 107) has an end wall (18, 118) opposite to the inlet opening (20, 120) the end wall (18, 118) having at least one outlet opening (8, 21, 121) and
e) at least one insert body (22) is arranged inside the through-flow element (7b, 107) the insert body (22) having sterilizing effect and traversing the cross section of the main body infusion channel given in the direction of flow.

2. Connector device according to claim 1, **characterized in that** the attachment body infusion channel (16, 116) tapers in the direction of flow at least in an inlet part (17, 117) surrounding an end piece of the through-flow element (7b, 107).

3. Connector device according to one of the preceding claims, **characterized in that** the outer side of the circumferential wall (19, 119) of the through-flow element (7b, 107) is cylindrical.

4. Connector device according to one of claims 1 or 2, **characterized in that** the outer side of the circumferential wall (19, 119) of the through-flow element (7b, 107) is conical with a diameter decreasing towards the end wall (18, 118).

5. Connector device according to one of the preceding claims, **characterized in that** the attachment body (10, 110) has a sterilizing effect, at least on a section of the attachment body infusion channel (16, 116).

6. Connector device according to one of the preceding claims, **characterized in that** the main body (1, 101) is integral.

7. Connector device according to one of the preceding claims, **characterized in that** the through-flow element (7b, 107) consists of a massive material with sterilizing effect.

8. Connector device according to one of the preceding claims, **characterized in that** the entire main body (1, 101) consists of a massive material with a sterilizing effect.

9. Connector device according to claim 7 or 8, **characterized in that** the massive material comprises a matrix of a plastic material, the matrix being doped with a sterilizing substance.

10. Connector device according to one of the preceding claims, **characterized in that** the attachment body (10, 110) has, at its end facing away from the main body (1, 101), a coupling piece for connection to a cannula, in particular a butterfly cannula.

11. Connector device according to one of claims 1 to 9, **characterized in that** the attachment body (10, 110) is part of a cannula, in particular a wing cannula.

## Revendications

1. Dispositif de connexion pour un cathéter, en particulier un cathéter veineux périphérique, comprenant
a) un corps principal (1, 101) comportant un canal de perfusion de corps principal (2, 102) et un élément d'écoulement (7b, 107) ayant un effet stérilisant et formant au moins une partie du canal de perfusion de corps principal (2, 102), dans lequel l'élément d'écoulement (7b, 107) comprend une lumière d'entrée (20, 120) et une paroi périphérique étanche aux liquides (19, 119), et
b) un corps de connexion (10, 110) ayant un canal de perfusion de corps de connexion (16, 116) et fixé de manière amovible au corps principal (1, 101), ,
**caractérisé en ce que**
c) lorsque le corps de connexion (10, 110) est fixé au corps principal (1, 101), l'élément d'écoulement (7b, 107) fait un ajustage serré étanche directement avec le canal de perfusion de corps de connexion (16, 116),
d) l'élément d'écoulement (7b, 107) a une paroi d'extrémité (18, 118), en face de la lumière d'entrée (20, 120), avec au moins une lumière de sortie (8, 21, 121) et
e) au moins un corps d'insert (22) à action stérilisante, traversant la section transversale du canal de perfusion de corps principal donnée dans la direction d'écoulement, est disposé à l'intérieur de l'élément d'écoulement (7b, 107).

2. Dispositif de connexion selon la revendication 1, **caractérisé en ce que** le canal de perfusion de corps de connexion (16, 116) au moins dans une pièce d'entrée (17, 117) entourant un embout de l'élément d'écoulement (7b, 107) se rétrécit dans la direction du fleuve.

3. Dispositif de connexion selon l'une des revendications précédentes, **caractérisé en ce que** la face extérieure de la paroi périphérique (19, 119) de l'élément d'écoulement (7b, 107) est cylindrique.

4. Dispositif de connexion selon l'une des revendications 1 ou 2, **caractérisé en ce que** la face extérieure de la paroi périphérique (19, 119) de l'élément d'écoulement (7b, 107) est conique avec un diamètre décroissant vers la paroi d'extrémité (18, 118).

5. Dispositif de connexion selon l'une des revendications précédentes, **caractérisé en ce que** le corps de connexion (10, 110) a un effet stérilisant au moins sur un trajet partiel dans le canal de perfusion de corps de connexion (16, 116).

6. Dispositif de connexion selon l'une des revendications précédentes, **caractérisé en ce que** le corps principal (1, 101) est monobloc.

7. Dispositif de connexion selon l'une des revendications précédentes, **caractérisé en ce que** l'élément d'écoulement (7b, 107) est constitué d'un matériau solide à effet stérilisant.

8. Dispositif de connexion selon l'une des revendications précédentes, **caractérisé en ce que** tout le corps principal (1, 101) est constitué d'un matériau solide à effet stérilisant.

9. Dispositif de connexion selon la revendication 7 ou 8, **caractérisé en ce que** le matériau solide comprend une matrice en matière plastique, la matrice étant dopée avec une substance stérilisante.

10. Dispositif de connexion selon l'une des revendications précédentes, **caractérisé en ce que** le corps de connexion (10, 110), à son extrémité éloignée au corps principal (1, 101), comporte une pièce d'accouplement destinée à être connectée à une canule, en particulier à une canule à ailes.

11. Dispositif de connexion selon l'une des revendications 1 à 9, **caractérisé en ce que** le corps de connexion (10, 110) fait partie d'une canule, en particulier d'une canule à ailettes.
